# EUROPEAN PATENT APPLICATION

(11) **EP 4 112 120 A1**
(43) Date of publication of application: **04.01.2023**
(21) Application number: 21761866.9
(22) Date of filing: 12.01.2021
(51) Int. Cl.: A61N 2/02, A61N 2/00, A61B 5/00

(54) **APPARATUS AND SYSTEM FOR GENERATING ALTERNATING MAGNETIC FIELD FOR DISEASE TREATMENT**

(30) Priority: 25.02.2020 KR 20200023042
(71) Applicant: Seoul National University R & DB Foundation, Seoul 08826 (KR); Brain & Beyonds Co., Ltd., Seoul 03080 (KR)
(72) Inventor: PAEK, Sun Ha, Seoul 04422 (KR); SEO, Young Deug, Daegu 41402 (KR)
(74) Representative: Algemeen Octrooi- en Merkenbureau B.V.
(86) International application number: PCT/KR2021/000422
(87) International publication number: WO 2021/172742

(57) **Abstract**

The technology disclosed in the present specification is characterized in that it provides an apparatus and system for generating an alternating magnetic field for disease treatment that are significantly safer and have fewer side effects compared to conventional methods of treating tumors by using magnetic nanoparticles. The apparatus for generating the alternating magnetic field may further include a ferrite core at the outside the coil, through which a more uniform and dense alternating magnetic field may be applied. The technology disclosed in the present specification is characterized by providing an AC magnetic field generating device and system for disease treatment that is significantly safer and has fewer side effects compared to the conventional method of treating tumors using magnetic nanoparticles.

## Description

### [Technical Field]

The technology disclosed herein relates to an apparatus and system for generating an alternating magnetic field for disease treatment, and more particularly, an apparatus and system for generating an alternating magnetic field that may reduce a size of a tumor by applying an alternating magnetic field of a s-pecific frequency to a target area of the body in order to treat diseases such as tumors in the body.

### [Background Art]

In the treatment of malignant or benign tumors, methods such as tumor tissue removal surgery, radiation therapy, and chemotherapy are widely used, but these treatment methods cause side effects that are harmful to the body simultaneously with the treatment of the tumor. Specifically, when a boundary between the tumor tissue and the normal tissue is unclear, it may not be possible to completely remove the tumor tissue with surgery, and in the case of radiation therapy, side effects such as hair loss occurs because the radiation therapy affects normal cells that proliferate. In addition, even in the case of chemotherapy, since complex side effects such as dizziness, vomiting, and decreased physical strength may be caused, there is a need for a treatment method capable of minimizing these side effects.

Therefore, when magnetic nanoparticles are administered to a target area in which the tumor is located and an alternating magnetic field is applied to the corresponding area, heat is generated from the magnetic nanoparticles that react to the alternating magnetic field, and in this case, an apparatus for a treatment method that reduces a size of the tumor through this exothermic effect has been conventionally used. However, when magnetic nanoparticles such as iron oxide particles are administered into the human body, they cannot be recovered and there is a possibility that another fatal side effect may be caused, and even in appearance, it is unavoidable that the corresponding area is darkly colored due to properties of the magnetic nanoparticles.

Techniques for methods of treating tumors are a task that needs to be first solved in modern society based on the expectations of people who want to maintain a healthy life, so that the need for the development of an apparatus that minimizes side effects to the human body in the process of treating various diseases is growing. Accordingly, the technology of implementing an alternating magnetic field generator that provides a therapeutic effect without the use of magnetic nanoparticles to solve the problems of the existing radiation therapy or of the alternating magnetic field generator using nanoparticles and to minimize side effects on the human body is constantly requested.

### [Disclosure]

### [Technical Problem]

The technology disclosed herein provides an apparatus for generating an alternating magnetic field that generates an alternating magnetic field that provides a therapeutic effect on a tumor without the use of magnetic nanoparticles in order to minimize side effects in the treatment of diseases.

In addition, the technology disclosed herein provides an apparatus for generating an alternating magnetic field provided with a plurality of coil heads having different diameters so as to apply an alternating magnetic field to various body parts.

In addition, the technology disclosed herein provides a system for generating an alternating magnetic field that includes an apparatus for generating an alternating magnetic field, that sets a frequency and strength of the alternating magnetic field through a controller, in which a current generator generates an alternating current having a set frequency and strength, and in which an alternating current flows through the coil to generate an alternating magnetic field in a target area of the body.

The technical objective to be achieved by the apparatus and system for generating the alternating magnetic field and system according to the technical concept of the technology disclosed in the present specification is not limited to the task for solving the above-mentioned problems, and another task that is not mentioned will be clearly understood by one of ordinary skill in the art through the description below.

### [Technical Solution]

An apparatus for generating an alternating magnetic field for disease treatment according to an embodiment of the technology disclosed in the present specification includes: a coil head configured to surround a target area of a body to generate an alternating magnetic field therein; a current generator configured to receive power from a power source and apply an alternating current to the coil head; and a controller configured to adjust a frequency and a strength of the alternating magnetic field, wherein the coil head includes a coil, and the coil is disposed in a plurality of parallel structures.

In addition, the coil may be provided with a ferrite core at the outside thereof.

The ferrite core may be formed to shield an outer surface of the coil.

The frequency of the alternating magnetic field generated in the coil head may be 80 KHz to 120 KHz.

In addition, an apparatus for generating an alternating magnetic field for disease treatment according to another embodiment of the technology disclosed in the present specification includes a heat reduction device at the outside of the coil head.

In the apparatus for generating the alternating magnetic field for the disease treatment according to another embodiment of the technology disclosed in the present specification, the coil head is configured to have a plurality of different inner diameters according to a target area of the body.

In addition, an apparatus for generating an alternating magnetic field for disease treatment according to another embodiment of the technology disclosed in the present specification includes a visual sensor that visually senses the target area of the body.

In addition, a system for generating an alternating magnetic field for disease treatment according to another embodiment of the technology disclosed in the present specification includes any one of the apparatuses for generating the alternating magnetic field according described above, wherein the system is configured to set the frequency and strength of the alternating magnetic field through the controller and for the current generator to generate the alternating current having the set frequency and strength, and is configured to allow the alternating current to flow through the coil to generate the alternating magnetic field in the target area of the body.

### [Advantageous Effects]

According to the apparatus for generating the alternating magnetic field for the disease treatment according to the embodiment of the technology disclosed in the present specification, it is possible to obtain a therapeutic effect with reduced side effects such as reducing a size of a tumor by applying only an alternating magnetic field to a target area of the body without the use of magnetic nanoparticles.

According to the apparatus for generating the alternating magnetic field according to the embodiment of the technology disclosed in the present specification, compared with the use of coils of a conventional series structure, by configuring a plurality of coils in a parallel structure, it is possible to significantly reduce heat generated in the apparatus for generating the alternating magnetic field to increase stability of the apparatus.

In addition, according to the embodiment of the technology disclosed in the present specification, by varying an inner diameter of a coil of the apparatus for generating the alternating magnetic field according to various parts of the body, it is possible to obtain an apparatus for generating an alternating magnetic field that may be applied to any part, and it is possible to maximize convenience by using a portable apparatus with a small size.

In addition, according to the embodiment of the technology disclosed in the present specification, by having a ferrite core that shields the outside of a coil, it is possible to apply a constant and uniform magnetic field to a target area, and it is possible to prevent unexpected side effects by using a low frequency harmless to the human body.

Meanwhile, the effects that may be achieved by the apparatus for generating the alternating magnetic field for the disease treatment according to the embodiment of the technology disclosed in the present specification are not limited to those mentioned above, and other effects not mentioned may be clearly understood to a person of an ordinary skill in the art by using the following description.

### [Description of Drawings]

A brief description of the drawings will be provided to more sufficiently understand the drawings which are used in the present specification.
FIG. 1 illustrates an entire apparatus for generating an alternating magnetic field as an embodiment of the technology disclosed in the present specification.
FIG. 2 illustrates a parallel structure of a plurality of coils included inside a coil head of the technology disclosed in the present specification.
FIG. 3A to FIG. 3D illustrate a state in which a ferrite core of the technology disclosed in the present specification shields the outside of a coil, and magnetic force lines by an alternating magnetic field.
FIG. 4 illustrates a detailed graph of an tumor volume reduction therapeutic effect of the technology disclosed in the present specification.
FIG. 5 visually illustrates a change in a size of a tumor of a test subject according to whether magnetic nanoparticles or not and whether an alternating magnetic field is applied with respect to a tumor volume reduction therapeutic effect of the technology disclosed in the present specification.
FIG. 6 illustrates a detailed graph of a tumor volume reduction effect that may be confirmed by using an apparatus for generating an alternating magnetic field of the technology disclosed in the present specification according to whether magnetic nanoparticles or not and whether an alternating magnetic field is applied.
FIG. 7 illustrates results of confirming a tumor treatment effect according to an embodiment of the technology disclosed in the present specification through various markers.
FIG. 8 illustrates a detailed graph of experimental results based on a tumor treatment effect according to an embodiment of the technology disclosed in the present specification.
FIG. 9 illustrates an embodiment of the technology disclosed in the present specification.
FIG. 10 illustrates another embodiment of the technology disclosed in the present specification.

### [Mode for Carrying out the Invention]

Since the technology disclosed in the present specification may be variously modified and have various forms, embodiments will be illustrated and described in detail in the following. This, however, by no means restricts the technology disclosed in the present specification to the specific embodiments, and it is to be understood as embracing all included in the spirit and scope of the technology disclosed in the present specification changes, equivalents, and substitutes.

In describing the technology disclosed in the present specification, when it is determined that a detailed description of the well-known art associated with the present invention may obscure the gist of the technology disclosed in the present specification, it will be omitted. Numbers (for example, first, second, etc.) to be used in the process of the description of the present specification are only identification codes for distinguishing one constituent element from other constituent elements.

In the present specification, it is to be understood that when one component is referred to as being "connected" or "coupled" to another component, it may be connected or coupled directly to the other component or may be connected or coupled to the other component by having another component intervening therebetween, particularly if there is no contrary description.

In addition, as for constituent elements represented by '-part, portion, or unit' in the present specification, two or more constituent elements may be combined into one constituent element, or one constituent element may be divided into two or more for each more subdivided function. In addition, each of constituent elements to be described below may additionally perform some or all of functions of other constituent elements in addition to a main function each constituent element is responsible for, and some of main functions that respective constituent elements are responsible for may also be exclusively performed by other constituent elements.

An expression such as "first" and "second" used in various embodiments may indicate various constituent elements regardless of order and/or importance, and does not limit corresponding constituent elements. For example, a first constituent element may be referred to as a second constituent element without deviating from the scope of the technology disclosed in the present specification and, similarly, a second constituent element may be referred to as a first constituent element.

Hereinafter, an apparatus for generating an alternating magnetic field for disease treatment according to a preferred embodiment will be described in detail.

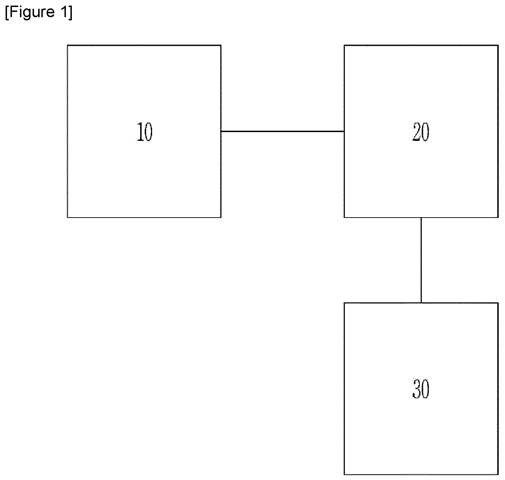

FIG. 1 illustrates an entire apparatus for generating an alternating magnetic field according to an embodiment of the present invention.

Referring to FIG. 1, an apparatus for generating an alternating magnetic field according to an embodiment of the present invention includes a coil head 10 including a coil that generates an alternating magnetic field and a current generator 20 that receives power from a power source and converts a direct current into an alternating current in order to apply an alternating current to the coil head 10, and it further includes a controller 30 configured to adjust a frequency and a strength of a magnetic field of the alternating current generated from the current generator 20.

The coil head 10 includes a coil 110 therein. When the alternating current applied from the current generator 20 flows through the coil 110, a substantially uniform alternating magnetic field is formed inside the coil 110. By applying such an alternating magnetic field to a target area of the body, an effect of reducing a volume and a size of a tumor may be obtained.

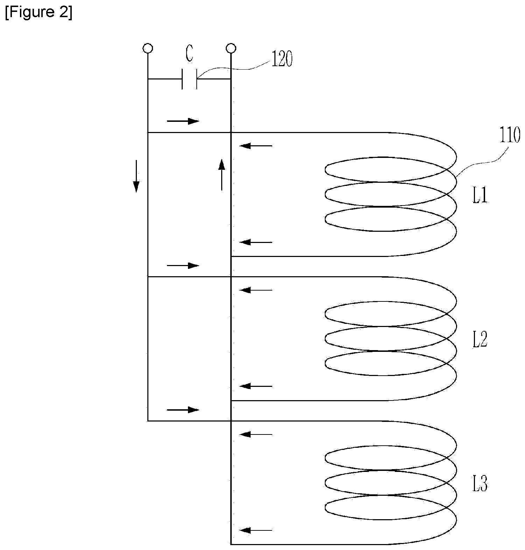

FIG. 2 illustrates a circuit in which the coil 110 according to an embodiment of the present invention is configured in a plurality of parallel structures.

Referring to FIG. 2, each coil 110 has a plurality of parallel structures, not a single series structure. The coil configuration of such a parallel structure significantly reduces heat generation of the apparatus for generating the alternating magnetic field according to the present embodiment, thereby increasing stability of the apparatus. Specifically, when the strength of the current is set to 1.8 A and the frequency of the alternating magnetic field is set to 98 KHz in the circuit of FIG. 2, temperature measurement results as shown in Table 1 below may be obtained.

**[Table 1]**

| **Coil structure** | **Before operation** | **After operation** |
|---|---|---|
| Serial | 26 °C | 97 °C |
| Parallel | 26 °C | 33 °C |

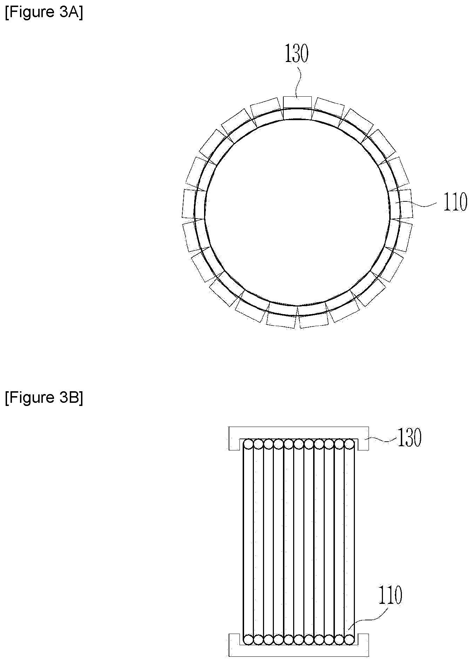

By configuring the coil in a plurality of parallel structures, it is possible to facilitate the circuit configuration thereof by lowering a voltage across both ends of a capacitor 120, and since voltages applied to the capacitor 120 and the coil 110 are very low compared to the circuit configured in series, it is possible to use an apparatus for generating an alternating magnetic field that is safer and significantly lowers heat generation. FIG. 3A to FIG. 3D illustrate that a ferrite core 130 is coupled to the coil 110 according to the embodiment of the present invention.

Referring to FIG. 3A, this is a view of the coil 110 in a circularly wound form from the side, and the ferrite core 130 surrounds the coil 110 in a shape that may shield all of the outside of the coil 110. FIG. 3B is a view of the shape of the ferrite core 130 surrounding the coil 110 from the front, and the ferrite core 130 shields all parts except the inside of the circular coil 110.

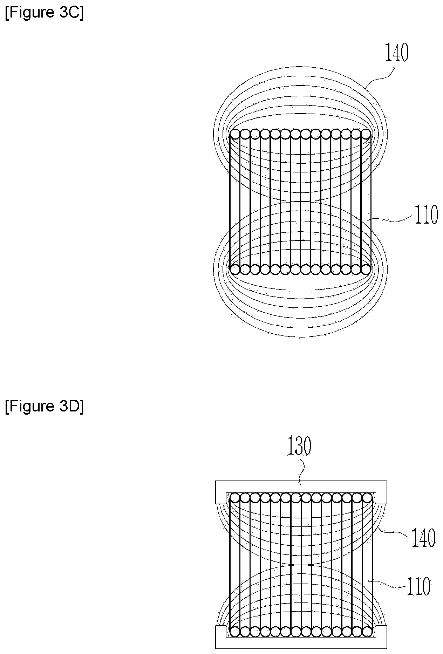

Therefore, unlike a magnetic force line 140 of an alternating magnetic field illustrated in FIG. 3C, a magnetic force line 140 of an alternating magnetic field of FIG. 3D having the ferrite core 130 at the outside of the coil 110 is a form in which the magnetic field is applied only to the inside. As the alternating magnetic field is concentrated inside, the magnetic field is further amplified, and it can be confirmed that this increases the magnetic field amplification magnitude more efficiently compared to installation of a simple straight-shaped ferrite core.

The ferrite core 130 in the present embodiment is made of a polycrystalline material sintered with materials such as zinc, manganese, iron oxide, and nickel, and has a large magnetic permeability and a large electrical resistance. Accordingly, the alternating magnetic field generated by the current is further amplified in size by the ferrite core 130 toward the inside, and the magnetic field applied to the outside of the ferrite core 130 may be shielded.

In a method of treating a tumor by applying an alternating magnetic field, there is a need to further increase the stability and reliability of the treatment method by applying a constant and uniform alternating magnetic field to a target area of the body. Accordingly, the embodiment of the present invention provides the apparatus for generating the alternating magnetic field that may apply a more uniform and concentrated alternating magnetic field to the target area of the body by utilizing the coil 110 to which the ferrite core 130 is added and that may effectively reduce a size of a tumor in a shorter time due to such an excellent effect.

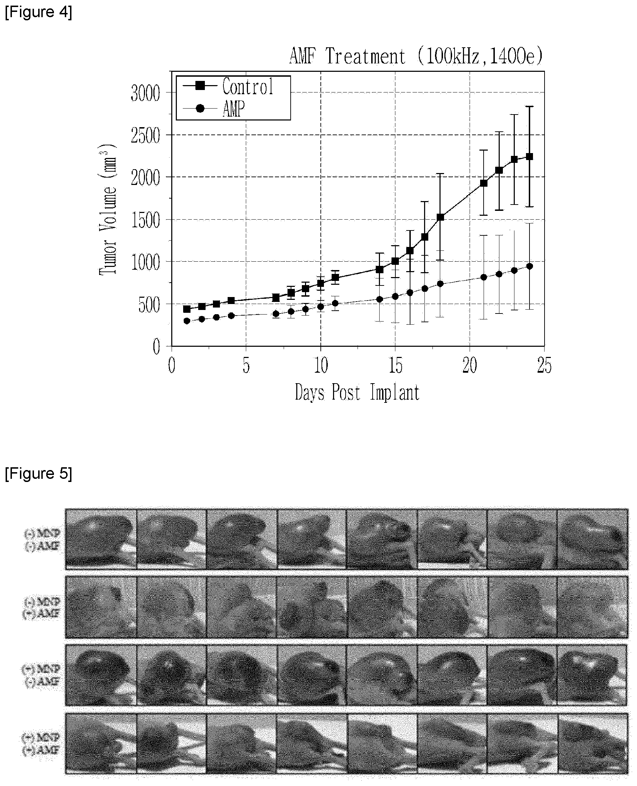

FIG. 4 illustrates a graph of an experiment result in which an alternating magnetic field was applied to a tumor, which is a target area of a test subject, under the same conditions for about 24 days by using the apparatus for generating the alternating magnetic field according to the embodiment of the present invention.

According to FIG. 4, for a tumor whose volume has significantly increased by proliferating tumor cells in the test subject, the experimental result of applying an alternating magnetic field for 30 minutes each time by setting the strength of the magnetic field to 140 Oe and the frequency thereof to 100 KHz can be confirmed. According to the graph, an alternating magnetic field was applied under the same conditions for 24 days, and the experimental group to which the alternating magnetic field was applied is shown as a circular dot, and it can be confirmed that the tumor size increased in a very small increase compared to the control group to which no alternating magnetic field was applied at all. This shows a result confirming that the cell division rate is rapidly reduced by applying the alternating magnetic field to the tumor area, considering the nature of a malignant tumor in which cell division continues and rapidly progresses.

FIG. 5 illustrates experimental results of injecting tumor cells into the test subject, and varying whether magnetic nanoparticles or not and whether an alternating magnetic field is applied.

Referring to FIG. 5, the first row shows the test subject in which only tumor cells were injected and magnetic nanoparticles were not injected, and to which an alternating magnetic field was not applied. The second row shows the test subject in which only the alternating magnetic field was applied to the tumor cells. The third row shows the test subject in which only magnetic nanoparticles were injected into the tumor cells. The fourth row shows the test subject in which the magnetic nanoparticles were injected into the tumor cells and the alternating magnetic field was applied together. Referring to the experimental results of the rows, respectively, there are 8 types of test subjects along respective columns. The first row shows that since no operation was performed, cell division proceeded and the tumor size significantly increased. In the second row, it can be seen that when only an alternating magnetic field was applied to the target area of the test subject without injection of magnetic nanoparticles, the size of the tumor was significantly reduced compared to that of the test subject in the first row. The third row is the experimental result of injecting only magnetic nanoparticles, and it can be seen that the area where the magnetic nanoparticles are injected is darkly colored, and the size of the tumor in the corresponding area is rather increased. This indicates a result that may imply that when the magnetic nanoparticles are injected into the body or the test subject, it may cause other unpredictable side effects. In the fourth row, as a result of injecting the magnetic nanoparticles and applying both the alternating magnetic field, it can be seen that the area in which the magnetic nanoparticles are injected is slightly darkly colored, indicating that the size of the tumor is also significantly reduced.

Through the experimental results as described above, when an alternating magnetic field is applied to the magnetic nanoparticles, although the effect of reducing the size of the tumor through the heat effect can be confirmed, it was confirmed that many side effects caused by injecting the magnetic nanoparticles into the human body cannot be avoided. Therefore, as a treatment method that may significantly reduce the tumor size while reducing these side effects, it can be confirmed that the method of applying only an alternating magnetic field to a target area of the body is safer and has an excellent effect.

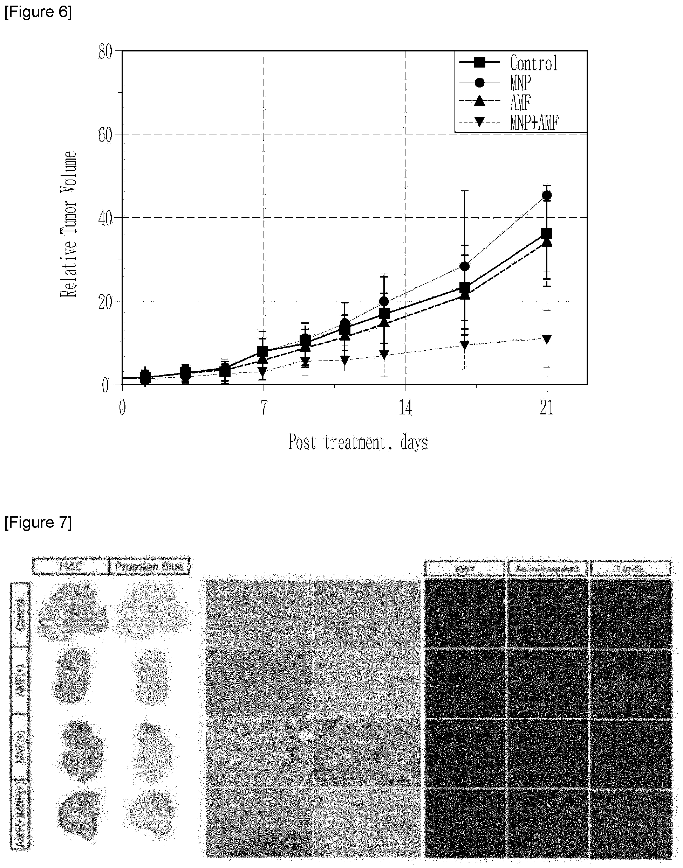

FIG. 6 illustrates a graph of experimental results under the same conditions as in FIG. 5.

According to FIG. 6, the square dot represents the tumor volume of the test subject without any change, the circular dot represents the tumor volume of the test subject injected with only magnetic nanoparticles, the triangular dot represents the tumor volume of the test subject to which an alternating magnetic field is applied, and the inverted triangular dot represents the tumor volume of the test subject injected with magnetic nanoparticles and applied with an alternating magnetic field.

Referring to the experimental results shown in FIG. 6, as shown visually confirming the change in the tumor volume of the test subject in FIG. 5, compared to the control group that did not perform any operation, it can be confirmed that the experimental group injected with only magnetic nanoparticles further increased the tumor volume, and in the remaining experimental group, the volume of the tumor is reduced in a certain part. Here, confirming the result of reducing the size of the tumor even though only the alternating magnetic field was applied without injecting the magnetic nanoparticles is a very dramatic achievement in the study of a safe tumor treatment method with few side effects to the human body.

FIG. 7 illustrates the experimental results using the apparatus according to the embodiment of the present invention, and illustrates the results of confirming the degree of cell division or apoptosis with the corresponding marker according to each experimental condition.

According to FIG. 7, it illustrates, for the same experimental conditions as in FIG. 5 and FIG. 6, the results of staining each cell tissue with H&E and Prussian Blue staining methods, and markers Ki67, Active-caspase3, and TUNEL to confirm the results for cell division and apoptosis. The H&E and Prussian Blue staining methods are widely used in medical diagnosis, and the composition of the tissue can be confirmed by biopsying a tissue suspected of cancer through pathology and staining a tissue section with H&E and Prussian Blue staining methods.

In addition, by confirming the distribution of the nuclear protein in the tumor cells through the marker Ki67, it is possible to verify how actively the cell division of the tumor cells is progressing through the experiment. On the contrary, the Active-caspase3 and TUNEL, as markers indicating apoptosis, indicate the progression of a suicide mechanism in which cells die by themselves, called apoptosis. Therefore, it can be interpreted that the more the marker is activated and displayed, the more the tumor cells decrease.

Referring back to FIG. 7, depending on the meaning of each marker, the control group can confirm the result of tumor cell division to some extent, and in the experimental group to which only the alternating magnetic field was applied (AMF(+)), cell division of tumor cells was significantly reduced, and apoptosis was also increased compared to the control group. In the experimental group (MNP(+)) injected with only the magnetic nanoparticles, it can be confirmed that the degree of cell division was similar to that of the control group, and it was observed at a level that apoptosis was not confirmed at all. Finally, in the experimental group (AMF(+)MNP(+)), in which the magnetic nanoparticles were injected and the alternating magnetic field was also applied, it can be confirmed that the degree of cell division was remarkably decreased and the apoptosis rate also increased most significantly.

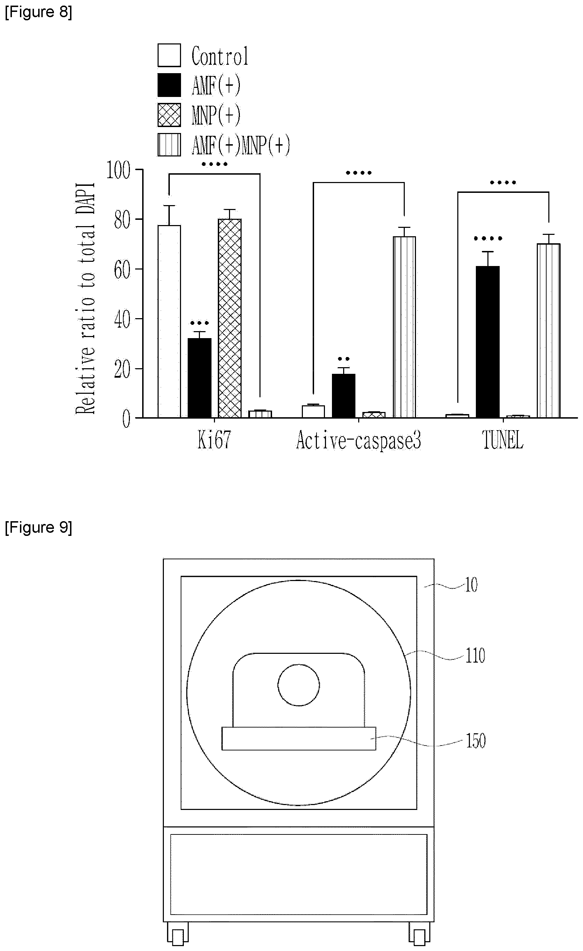

With reference to FIG. 7, FIG. 8 illustrates a graph of the experimental results through the corresponding markers in more detail.

According to FIG. 8, as confirmed in FIG. 7, it can be seen that Ki67, which means cell division, is significantly reduced even in the experimental group to which only the alternating magnetic field is applied, and even in the case of Active-caspase3 and TUNEL, which mean apoptosis, it can be seen that the result significantly increased in the experimental group to which only the alternating magnetic field was applied.

FIG. 9 illustrates an apparatus for generating an alternating magnetic field for disease treatment according to an embodiment of the present invention.

Referring to FIG. 9, it can be seen that an apparatus for generating an alternating magnetic field is formed so that the coil head 10 and the inner coil 110 thereof have an internal diameter large enough to cover a circumference of a person's body.

FIG. 9 illustrates that the apparatus for generating the alternating magnetic field further includes a bed part 150 capable of stably supporting a person's body in a lying position. The bed part 150 may be moved forward or backward to properly aim a target area of the body in order to apply the alternating magnetic field.

On the other hand, although not shown in FIG. 9, it may further include a ferrite core 130 that shields an outer surface of the coil 110, and may further include a controller 30 capable of adjusting a strength and frequency of the alternating magnetic field and a camera part.

The camera part may be installed in an inner area of the coil head, and may image a target area of the body and display it on various displays, and in addition, by further including a visual sensor together with the camera part, an area and volume change of the target area of the body may be detected and tracked.

FIG. 9 illustrates an apparatus for generating an alternating magnetic field according to another embodiment of the present invention.

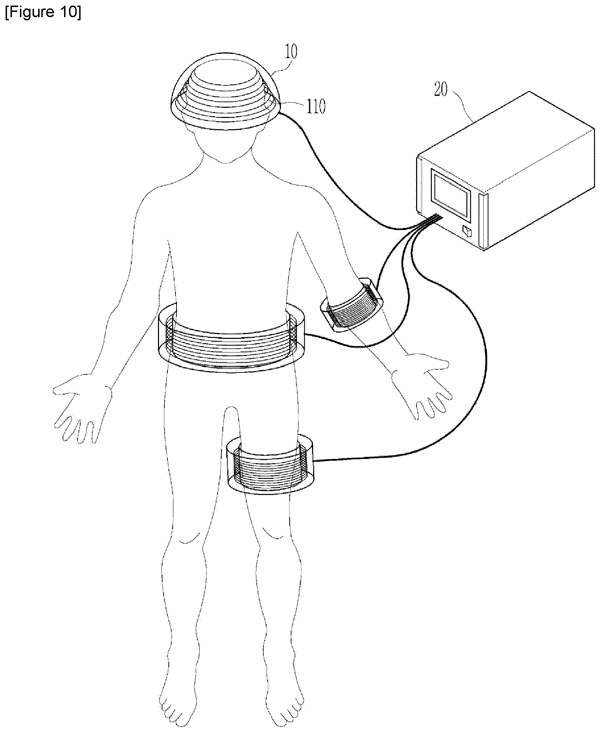

Referring to FIG. 10, a coil head 10 and a coil 110 of the present apparatus for generating an alternating magnetic field may be formed in a shape having various internal diameters so as to wrap various parts of the body.

As shown in FIG. 10, the coil head 10 and the coil 110 have diameters that fit the head, arm, torso, and leg parts, and their sizes are significantly reduced compared to that of the apparatus for generating the alternating magnetic field according to the embodiment of FIG. 9, and it can be seen that portability and convenience are further improved through the reduction in size. According to the present embodiment, the system for generating the alternating magnetic field that is provided with the portable current generator 20, the coil head 10, and a coil 110 and in which the current generator 20 is connected to the coil head 10 and the alternating magnetic field is generated as the alternating current flows to be designed to treat diseases by simply targeting the target area of the body, is provided.

Although the present invention has been described in detail above, the scope of the present invention is not limited thereto, and it will be apparent to those skilled in the art that various modifications and variations are possible without departing from the spirit of the present invention described in the appended claims.

### [Description of symbols]

10: coil head
20: current generator
30: controller
110: coil
120: condenser
130: ferrite core
140: magnetic force line
150: bed part

## Claims

1. An apparatus for generating an alternating magnetic field for disease treatment, comprising:
a coil head configured to surround a target area of a body to generate an alternating magnetic field therein;
a current generator configured to receive power from a power source and apply an alternating current to the coil head; and
a controller configured to adjust a frequency and a strength of the alternating magnetic field,
wherein the coil head includes a coil, and the coil is disposed in a plurality of parallel structures.

2. The apparatus for generating the alternating magnetic field for the disease treatment of claim 1, wherein
the coil is provided with a ferrite core at the outside thereof.

3. The apparatus for generating the alternating magnetic field for the disease treatment of claim 2, wherein
the ferrite core is formed to shield all of an outer surface of the coil.

4. The apparatus for generating the alternating magnetic field for the disease treatment of claim 1, wherein
the frequency of the alternating magnetic field generated in the coil head is 80 KHz to 120 KHz.

5. The apparatus for generating the alternating magnetic field for the disease treatment of claim 1, further comprising
a heat reduction device at the outside of the coil head.

6. The apparatus for generating the alternating magnetic field for the disease treatment of claim 1, wherein
the coil head has a plurality of different inner diameters according to a target area of the body.

7. The apparatus for generating the alternating magnetic field for the disease treatment of claim 1, further comprising
a visual sensor that visually senses the target area of the body.

8. A system for generating an alternating magnetic field for disease treatment, comprising:
the apparatus for generating the alternating magnetic field according to any one of claim 1 to claim 7,
wherein the system is configured to set the frequency and strength of the alternating magnetic field through the controller and for the current generator to generate the alternating current having the set frequency and strength, and is configured to allow the alternating current to flow through the coil to generate the alternating magnetic field in the target area of the body.
